# EUROPEAN PATENT SPECIFICATION

(11) **EP 2 320 831 B1**
(45) Date of publication and mention of the grant of the patent: **19.08.2020**
(21) Application number: 09789632.8
(22) Date of filing: 01.05.2009
(51) Int. Cl.: B29L 31/00, B29C 41/14, A61F 2/12

(54) **SOFT FILLED PROSTHESIS SHELL WITH DISCRETE FIXATION SURFACES**
WEICHE GEFÜLLTE PROTHESENSCHALE MIT DISKRETEN FIXIERFLÄCHEN
COQUE SOUPLE DE PROTHÈSE REMPLIE AVEC DES SURFACES DE FIXATION DISCRÈTES

(30) Priority: 13.08.2008 US 88427 P
(43) Date of publication of application: 18.05.2011
(73) Proprietor: ALLERGAN, INC., Irvine, CA 92612 (US)
(72) Inventor: VAN EPPS, Dennis, Goleta CA 93117 (US); POWELL, Thomas, Santa Barbara CA 93111 (US)
(74) Representative: Hoffmann Eitle
(86) International application number: PCT/US2009/042559
(87) International publication number: WO 2010/019292

(56) References cited:
- US-A- 3 600 718
- US-A1- 2003 205 846

## Description

### Field of the Invention

The present invention relates to soft prosthetic implants and, more particularly, to textured exterior surfaces of such implants, for instance, breast implants.

### Background of the Invention

Implantable prostheses are commonly used to replace or augment body tissue. In the case of breast cancer, it is sometimes necessary to remove some or all of the mammary gland and surrounding tissue, which creates a void that can be filled with an implantable prosthesis. The implant serves to support surrounding tissue and to maintain the appearance of the body. The restoration of the normal appearance of the body has an extremely beneficial psychological effect on post-operative patients, eliminating much of the shock and depression that often follows extensive surgical procedures. Implantable prostheses are also used more generally for restoring the normal appearance of soft tissue in various areas of the body, such as the buttocks, chin, calf, etc.

Soft implantable prostheses typically include a relatively thin and quite flexible envelope or shell made of vulcanized (cured) silicone elastomer. The shell is filled either with a silicone gel or with a normal saline solution. The filling of the shell takes place before or after the shell is inserted through an incision in the patient.

In the United States, women can choose between two different types of breast implant shell surfaces: a smooth surface and a textured surface. The surgeon generally recommends the type of surface based on his or her technique and the shape of the breast implant chosen to best fit the needs of each patient.

Breast implants are not without complications, one of which is termed capsular contracture. This is a complication that occurs upon contraction of a fibrous outer capsule that forms around the implant, which tends to render the implant spherical and stiff and aesthetically undesirable. According to the United States Food and Drug Administration's (FDA) Breast Implant Consumer Handbook (2004), the literature shows that textured surface breast implants may decrease the capsular contracture rate.

Texturing may be provided in a number of ways. Silicone gel breast implants covered with a thin layer of textured polyurethane foam enjoyed considerable popularity in the 1980s because of their remarkable resistance to the early development of fibrous capsular contracture.

A process for forming a textured surface implant using round salt crystals is disclosed in Powell et al., U.S. Patent Application No. 12/261,939 filed on October 30, 2008, and is entitled Soft Prosthesis Shell Texturing Method.

US 2003/0205846 discloses an implant showing a textured surface portion engaging tissue ingrowth in the body and a second portion being smooth.

US 4,955,907 discloses an implant for use in the human body. A textured covering can be provided, wherein the textured covering may form the entirety or only a portion of the covering of the implant.

US36700718 discloses an implant comprising a shell and a plurality of patches affixed to the back of said shell wherein the patches can be made of an open cell foam type material.

Despite many advances in the construction of soft prosthetic implant shells, there remains a need for a better method for texturing their outer surfaces to enhance adhesion, especially for shaped devices, without inciting capsular contracture, while at the same time not completely losing the benefits of smooth implants.

### Summary of the Invention

The present invention relates to a breast prosthesis as defined in the independent claims. Advantageous embodiments are recited in the dependent claims.

The present invention provides a prosthesis suitable for implantation in a human being, for example, a breast implant suitable for use in reconstruction or augmentation of the human breast. The prosthesis generally comprises a soft prosthetic implant shell, such as a silicone elastomer shell, that includes discrete fixation surfaces thereon for enhancing and/or controlling tissue ingrowth or adhesion. The prosthesis may further comprise a core, for example a gel core, encased by the shell. Alternatively, the shell may be structured to be suitable for filling, for example, with saline, after implantation of the prosthesis in a human being.

In accordance with one aspect of the invention, the fixation surfaces are surfaces on of an exterior of the shell having open cell textures that enhances and/or controls tissue ingrowth or adhesion relative to an otherwise identical surface without such texture, roughness or sheen.

In one embodiment of the invention, the fixation regions are positioned or configured such that the prosthesis after implantation in the body, moves more naturally with the human body, for example, in relative unity with the muscles of the body. Because the implants move more naturally with the human body, the present implants may be less prone to wear resulting from material stresses relative to conventional implants without such fixation regions.

More specifically, the fixation surfaces, hereinafter sometimes referred to as fixation regions, may be located at specific regions on an anterior face of the shell, that is, a face of the shell which faces the front of the human body when the implant has been appropriately implanted in the human body. Alternatively or additionally, one or more discrete fixation surface may be provided on a periphery of the shell (e.g. circumferentially) and/or on the posterior face of the shell, that is, the face of the shell that faces the back of the human body when the implant has been implanted in the human body.

In an even more specific aspect of the invention, the fixation regions comprise at least one elongated region located on the anterior surface of the shell. The at least one elongated region may be, for example, a band-shaped region or alternatively, a plurality of band shaped regions having enhanced texture, roughness or sheen.

The elongated fixation regions may be positioned to align with one of the pectoralis major muscle groups or pectoralis minor muscle groups of the human body when the implant is implanted in the body. For example, in one embodiment of the invention, the at least one elongated region comprises a diagonally positioned band shaped region intended to align with the pectoralis major muscle group when the implant has been implanted in the body. In another embodiment, the at least one fixation region comprises a plurality of elongated regions in a radiating configuration generally copying the positioning of the pectoralis minor muscle group wherein the implant has been implanted in the body.

In aspect of the invention, the prosthesis comprises a breast implant having a shell including a fixation region having a first texture comprising open cells and a balance of the shell surface having a second texture comprising open cells that are different from the ones of said first texture. In other words, in some embodiments of the invention, the entire, or substantially entire, exterior of the breast implant shell is a textured surface with specific regions thereof having a greater degree of texturing relative to the remaining portions of the textured surface.

It is contemplated that such different texturing will stimulate or encourage different degrees of tissue ingrowth or adhesion at the different fixation regions. For example, in one embodiment, the first fixation region is located on a posterior surface of the implant and the second fixation region is located on an anterior surface of the implant. The first fixation region may be defined by a texture that is more conducive to tissue interaction and adhesion whereas the second fixation region may be defined by a texture that is relatively less conducive to tissue interaction and adhesion.

In the present invention, the prosthesis comprises a shell having an exterior structured to contact tissue, the shell including a first fixation surface having a first open cell structure, and a second fixation surface having a second open cell structure different than said first open cell structure. In addition, the first fixation surface and the second fixation surface are positioned to encourage respectively different degrees of tissue ingrowth or tissue adhesion by the body at a body-shell interface.

The first open cell structure comprises relatively large open cells and the second open cell structure comprises relatively smaller open cells. Additionally, the first open cell structure may comprise a first distribution of cells and the second open cell structure may comprise a second distribution of cells wherein the first distribution of cells is relatively more dense than the second distribution of cells.

In yet another specific aspect of the invention, the first open cell structure comprises relatively large rounded open cells and the second open cell structure comprises relatively small rounded open cells. Alternatively, the first open cell structure comprises relatively rounded open cells and the second open cell structure comprises relatively angular open cells

Advantageously, in accordance with certain embodiments, the first and second fixation surfaces are positioned and structured to be at least somewhat effective to disrupt or disorient capsular tissue formation about the prosthesis after the prosthesis has been implanted in the body.

The present invention further provides a breast prosthesis shell for implantation in a human being, the shell manufactured by the steps of providing a shell precursor, applying a layer of silicone elastomer to the shell precursor, applying solid particles of a first configuration to a portion of the layer of silicone elastomer and applying solid particles of a second configuration to another portion of the layer of silicone elastomer before the layer is fully cured. After the layer including the solid particles embedded therein is cured, the solid particles are then dissolved, for example, by means of a solvent that does not dissolve the silicone elastomer to any appreciable extent. The resulting elastomer shell includes a first open cell texture region formed by said application of the solid particles of the first configuration, and a second open cell texture region formed by said application of the solid particles of the second configuration.

A further understanding of the nature and advantages of the present invention are set forth in the following description and claims, particularly when considered in conjunction with the accompanying drawings in which like parts bear like reference numerals.

### Brief Description of the Drawings

Features and advantages of the present invention will become appreciated as the same become better understood with reference to the specification, claims, and appended drawings wherein:
Figures 1A-1B are anterior or front and side elevational views of an exemplary round breast implant of the present invention having rear and peripheral fixation surfaces;
Figures 2A-2B are front and side elevational views of an exemplary shaped breast implant of the present invention having rear and peripheral fixation surfaces;
Figures 3A and 3B are schematic views calls a woman's upper torso showing, respectively, pectoralis major and pectoralis minor muscle positions on one side;
Figures 4A and 4B are vertical sectional views through a woman's breast and adjacent chest anatomy showing, respectively, subglandular and submuscular placement of a breast implant;
Figures 5A-5B are front and side elevational views of an exemplary round breast implant of the present invention having rear and peripheral fixation surfaces as well as a frontal band-shaped fixation surface; and
Figures 6A-6B are front and side elevational views of an exemplary shaped breast implant of the present invention having rear and peripheral fixation surfaces as well as frontal band-shaped fixation surfaces.
Figure 7 is a front elevational view of another breast implant in accordance with the invention including a first fixation region having a first texture and a second fixation region having a second texture different from the first texture.
Figures 8A and 8B are front and rear elevational views of an exemplary round breast implant of the present invention having a front texture and a rear texture that are different from one another.

### Detailed Description

The present invention provides a saline- or gel-filled soft implant shell, preferably a silicone elastomer shell, with a fixation surface over an exterior portion. The primary application for such soft implants is to reconstruct or augment the female breast. Other potential applications are implants for the buttocks, testes, or calf, among other areas.

The term fixation surface refers to a surface on the exterior of the implant shell adapted to encourage tissue ingrowth or adhesion. A fixation surface may be a roughened or textured area in comparison to other smooth or less textured areas of the implant shell. For example, a textured surface may be formed by a salt removal process, such as with the Allergan BIOCELL® surface. Other configurations of fixation surfaces include textured separate elements such as patches or films adhered to the outside of the implant, as well as a roughened surface features formed during the mold process. One exemplary method is to roughen the interior surface of the mold in which the implant shell is formed. Another method is to roughen the exterior of the implant after formation. The present invention should not be considered limited to any particular type of texturing or fixation surface, though there might be certain advantages with one or more of these techniques.

Previous breast implants have been formed with either all smooth or all textured shells. Some of the prior art designs also described placing textured surfaces on the posterior or rear of the implant to encourage adhesion to the chest wall. To date, particular placement of discrete fixation surfaces on the periphery or front side of breast implants has not been described in the art. The present invention illustrates a number of different configurations, but those of skill in the art will understand that other shapes and placements are possible, and that the invention should be limited only by the appended claims.

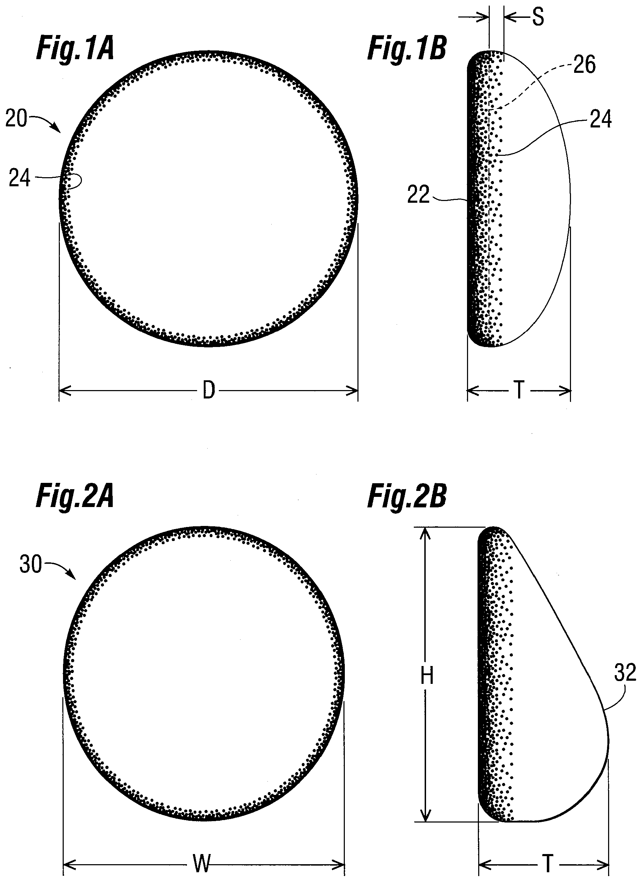

Figures 1A-1B are front and side elevational views of an exemplary round breast implant 20 of the present invention having a generally smooth exterior except for posterior or rear 22 and peripheral 24 fixation surfaces. Alternatively, the exterior surface of the implant may have less textured areas outside of the fixation surfaces 22, 24, such as a fine textured or matte finish, or a combination of smooth and less textured areas. Indeed, the fixation surfaces 22, 24 themselves may have differing degrees of texturing. In this embodiment, the fixation surfaces 22, 24 are illustrated with stippling, which is representative of either roughness from a salt removal process or from a roughened mold. Of course, the fixation surfaces 22, 24 may also be formed by other means, such as for example with a separate fabric or foam layer adhered to the implant 20. The diameter **D** and front-to-back thickness **T** of the implant are shown and vary depending on the patient's chest size and aesthetic considerations.

Desirably, the rear fixation surface 22 extends to the apex 26 or generatrix of the convex outer periphery of the implant 20. The peripheral fixation surface 24 continues forward a short distance **S** around the anterior or front surface of the implant. In a preferred embodiment, the distance **S** is between about 10-30% of the front-to-back thickness **T.** Preferably, the peripheral fixation surface 24 extends completely around the periphery of the implant 20, therefore rendering the round implant 20 completely axisymmetric. However, the peripheral fixation surface 24 may be abbreviated so as to extend around only a portion of the periphery of the implant, such as the inferior or superior half, or may be broken up into spaced segments. In one embodiment, the peripheral fixation surface 24 is broken up to evenly spaced segments resulting in alternating smooth and textured areas such that the implant 20 is substantially axisymmetric and the surgeon need not bother with any particular implant orientation.

Figures 2A-2B illustrate a shaped breast implant 30 of the present invention having an inferior frontal lobe 32 simulating a natural breast. The implant 30 includes a rear fixation surface 34 and a peripheral fixation surface 36, as in the embodiment of Figures 1A-1B. The width **W,** height **H,** and front-to-back thickness **T** of the implant are shown. If the front projection is round, then **W = H,** otherwise **W** may be greater than or less than **H.** When provided with a natural shape, the implant 30 has a proper orientation, namely with the inferior lobe 32 at the lower center. Accordingly, the peripheral fixation surface 36 may extend completely around the periphery of the implant, or may be formed in discrete areas and be oriented relative to the natural shape of the implant. For example, the peripheral fixation surface 36 may be formed only around the inferior or lower half of the implant, or may be formed only on the sides. Proper placement of the implant 30 based on its natural shape will therefore simultaneously properly place the discrete fixation surfaces.

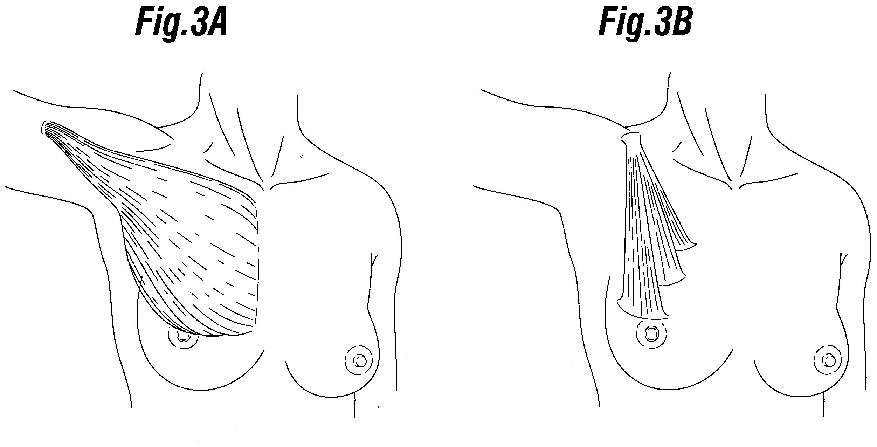

Figure 3A illustrates a woman's upper torso schematically showing on one side placement of the pectoralis major muscle group, while Figure 3B illustrates the pectoralis minor muscle group. These two most groups overlap one another and extend generally from the shoulder or collarbone region to the rib cage underneath the breast. Extension and contraction of these muscles is obviously quite important and movement of the armed, and therefore the area underneath the breast experiences a great deal of stretching and contracting along the lines of these muscle groups. As will be explained below, one aspect of the present invention is to provide fixation surfaces aligned with these muscle groups. The points or lines of contact of the implant with the primary chest muscles experience greater movement than other areas, and thus fixation surfaces coincident with or aligned with the muscles are more likely to remain secured (i.e., they move with the muscle). In contrast, placing a fixation surface away from a muscle group may be subject to greater shear forces from the nearby muscle.

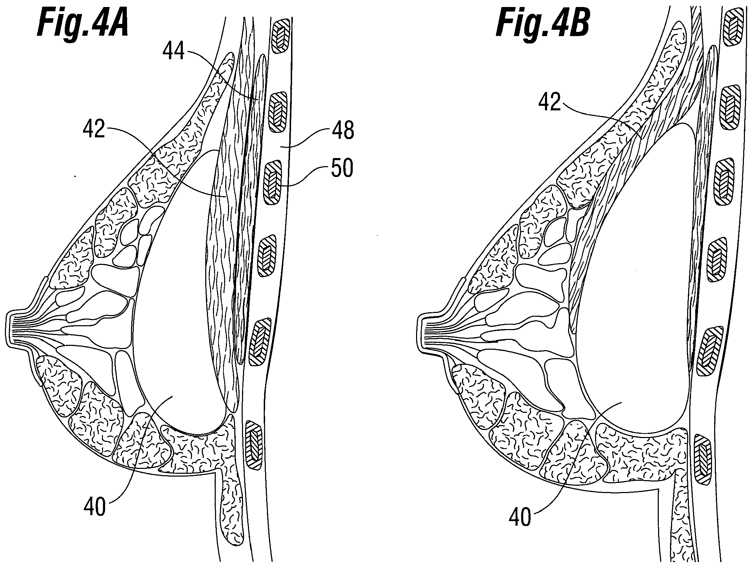

Figure 4A is a vertical sectional view through a woman's breast and adjacent chest anatomy showing a subglandular placement of a breast implant 40. The implant 40 is positioned over the top of the pectoralis major muscle group 42, which in turn overlays the pectoralis minor muscle group 44. The chest wall 48 showing a plurality of ribs 50 is also indicated underneath the pectoralis minor muscle 44. Figure 4B is a vertical sectional view as in Figure 4A but showing a submuscular placement of the implant 40, underneath the pectoralis major muscle group 42. Both these two implant placements are utilized primarily depending on the surgeon's clinical determination, sometimes influenced by a dialogue between patient and the surgeon and desired outcome. Depending on the implant placements, the implant 40 may be in contact with one or both muscle groups.

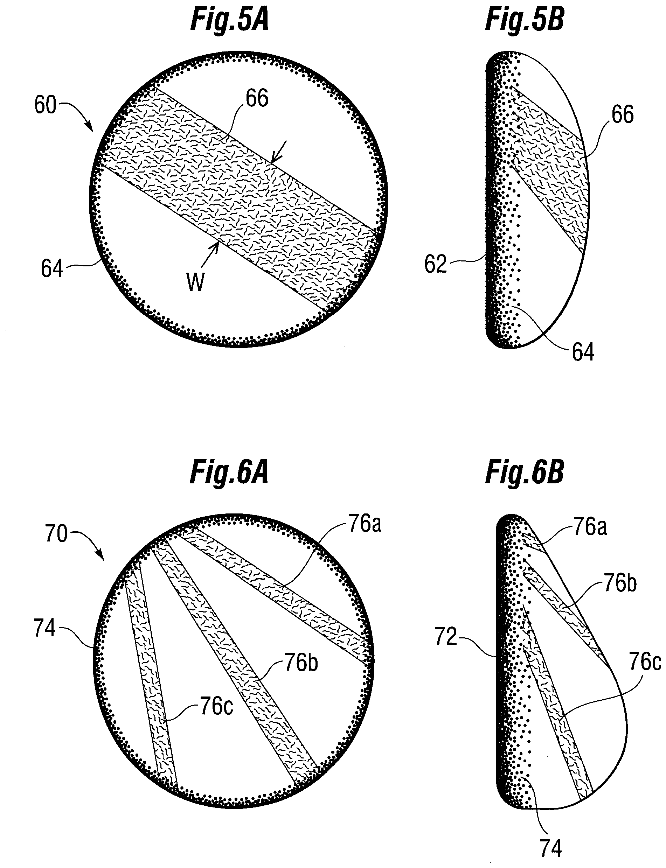

Figures 5A-5B are front and side elevational views of an exemplary round breast implant 60 of the present invention having a rear fixation surface 62, a peripheral fixation surface 64, and a frontal band-shaped fixation surface 66. The band-shaped fixation surface 66 extends generally along a diagonal angle and commences at the front border of the peripheral fixation surface 64. The illustrated embodiment, the fixation surface 66 has a constant width **W** as seen from the front in Figure 5A. In one embodiment, the width **W** is between about 2-15 mm. Alternatively, the front view of the fixation surface 66 may be other than a constant width, and may have non-linear boundaries.

In a preferred embodiment, the band-shaped fixation surface 66 is generally oriented with either the pectoralis major or pectoralis minor muscle groups. For instance, if the implant 60 is destined for a submuscular placement such as in Figure 4B, the fixation surface 66 may be oriented to be generally aligned with the pectoralis major muscle group, as seen in Figure 3A. Alternatively, the angle at which the insertion surface 66 is oriented may be an approximation of the average angle of the pectoralis major and pectoralis minor muscle groups. In this way, the implant 60 has a fixation surface 66 to encourage tissue ingrowth or adhesion along the major stress lines of the implant. Preferably, the fixation surface 66 is angled between about 30-60° with respect to a vertical plane through the implant 60. Of course, if the implant 60 is round as shown, the fixation surface 66 itself defines the orientation thereof. In one embodiment, the band-shaped fixation surface 66 is centered about the center of the implant 60, therefore creating two symmetric orientations 180° apart. This arrangement facilitates implant by providing two possible orientations for the surgeon.

The band-shaped fixation surface 66 is shown as having a different composition than either the rear fixation surface 62 or peripheral fixation surface 64. In this respect, any of the different fixation surfaces described herein may be formed in the same way, or using different techniques. For instance, the fixation surfaces 62, 64 may be texturing in the implant shell, while the band-shaped fixation surface 66 is a separate element such as a patch or film adhered to the front surface of the implant. The reader will understand that all combinations of the various fixation surface inclusions, placements and types are contemplated. Likewise, any of these potential combinations may be provided on any of the various round or shaped implants as shown, or others not shown. For instance, fixation surfaces may also be useful for implants for the buttocks, testes, or calf, among other areas, and may be aligned with muscle groups in those areas.

Figures 6A-6B illustrate an exemplary shaped breast implant 70 of the present invention. The implant 70 again features a rear fixation surface 72, a peripheral fixation surface 74, and a plurality of separate band-shaped fixation surfaces 76a, 76b, 76c. These discrete fixation surfaces 76a, 76b, 76c desirably mimic one or more of the muscle groups described above. For example, the three fixation surfaces 76a, 76b, 76c may be generally oriented relative to the fan-shaped pectoralis minor muscle group. Because the shaped implant 70 is orientation-specific, proper placement of the implant automatically orients the fixation surfaces 76a, 76b, 76c with the particular muscle group. As mentioned above, the various fixation surfaces 72, 74, 76a, 76b, and 76c may be formed with a similar level of roughness, or some may be less textured, such as with a matte finish. For instance, the rear and peripheral fixation surfaces 72, 74 may have a fine, matte finish, while the frontal fixation surfaces 76a, 76b, 76c are more densely textured. The present invention contemplates all permutations of texturing choices.

In cross-section, the textured implant shells of the present invention may be single- or multi-layered. The overall thickness of the textured implant shell wall may be somewhat greater than a similar smooth-walled shell because of the extra layers of texture.

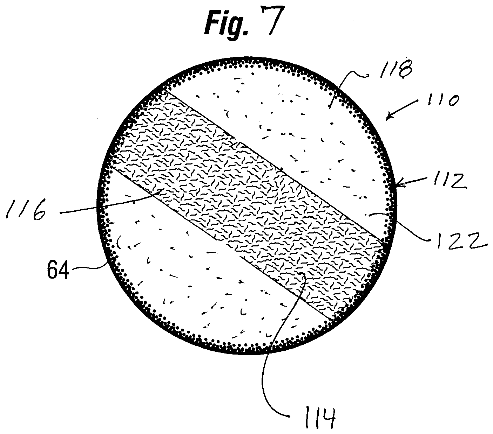

Turning now to Fig. 7, an anterior (front) view of another breast implant of the present invention is shown generally at 110. The implant 110 includes a shell 112 having an exterior surface including a first fixation region 114 having a first texture 116 and a second fixation region 118 having a second texture 122 that is different from the first texture 116. In the shown embodiment, the first texture 116 is a more "aggressive" texture than the second texture 122. The first texture 116 is structured to encourage a greater degree of tissue interaction than the second texture 122.

In lieu of the second texture 122, it is contemplated that the second fixation region 118, and perhaps the entire balance of the exterior of the shell 112, may be a low sheen surface, for example a matte finish, which encourages some tissue interaction which is less than that of a low textured surface.

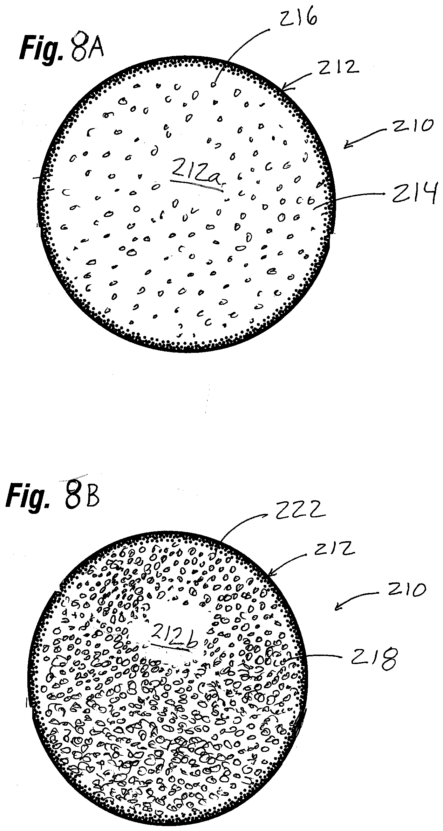

Turning now to Figs. 8A and 8B, anterior (front) and posterior (rear) views, respectively, of another breast implant in accordance with the invention are shown generally at 210. The implant 210 includes a shell 212 having an anterior surface 212a and a posterior surface 212b, and including a second fixation region 214 having a second texture 216 and a first fixation region 218 having a first texture 222 that is different from the second texture 216. In the shown embodiment, the second texture 216 may encompass the entire, or substantially entire, anterior surface 212a of the implant 210. The second texture 216 is defined by a second distribution of pores, crevices or caverns that is relatively less dense than that of the first texture 222. The first texture 222, which may encompass the entire, or substantially entire, posterior surface 221b of the implant 210, is structured to encourage a greater degree of tissue interaction and adhesion than that of the second texture 216.

The shells 112 and 212 may be manufactured by a method of the invention comprising the steps of providing a shell precursor; applying a layer of silicone elastomer to the shell precursor, applying solid particles of a first configuration to a portion of the layer of silicone elastomer and applying solid particles of a second configuration to another portion of the layer of silicone elastomer before the layer is fully cured. After the layer including the solid particles embedded therein is cured, the solid particles are then dissolved, for example, by means of a solvent that does not dissolve the silicone elastomer to any appreciable extent. The resulting elastomer shell includes a first open cell texture region formed by said application of the solid particles of the first configuration, and a second open cell texture region formed by said application of the solid particles of the second configuration.

One process for forming flexible implant shells for implantable prostheses involve dipping a suitably shaped mandrel into a silicone elastomer dispersion. Many such dispersions are used in the field. Basically they contain a silicone elastomer and a solvent. The silicone elastomer is typically polydimethylsiloxane, polydiphenyl-siloxane or some combination of these two. Typical solvents include xylene or 1,1,1-trichloroethane. Different manufacturers vary the type and amount of the ingredients in the dispersion, the viscosity of the dispersion and the solid content of the dispersion. Nonetheless, the present invention is expected to be adaptable to have utility with a wide variety of silicone rubber dispersions.

The mandrel is withdrawn from the dispersion and the excess silicone elastomer dispersion is allowed to drain from the mandrel. After the excess dispersion has drained from the mandrel at least a portion of the solvent is allowed to volatilize or evaporate. Normally this is accomplished by flowing air over the coated mandrel at a controlled temperature and humidity. Different manufacturers use various quantities, velocities or directions of air flow and set the temperature and humidity of the air at different values. However, the desired result, driving off the solvent, remains the same.

It is also common for prostheses manufacturers to repeat this dip and volatilize procedure a number of times so that a number of layers are built up on the mandrel to reach a desired shell thickness. A layered structure like most current silicone elastomer shells can be made by sequentially dipping the mandrel in different dispersions. Alternatively, the steps may be repeated in a single dispersion so that the finished product is a single homogenous material or layer. That is, the dipping process may be done in multiple stages or steps, each step adding more material, yet the finished product exhibits no distinct layers and the entire shell wall is homogenous or uniform in composition.

An exemplary process for forming the fixation surfaces on either a multi-layered shell or a single-layered shell will now be described. After the mandrel is raised out of the dispersion with what is to be the final layer adhering thereto, this layer is allowed to stabilize. That is, it is held until the final coating no longer flows freely. This occurs as some of the solvent evaporates from the final coating, raising its viscosity.

Again, it should be understood that alternative methods are contemplated for forming the flexible shell prior to the texturing process. The dip molding process advantageously results in the flexible shell pre-mounted on a dipping mandrel, which can then be used for the texturing process. However, if the flexible shell is made by another technique, such as by rotational molding, it can subsequently be mounted on a dipping mandrel and the process continued in the same manner.

Once the flexible shell has been stabilized and mounted on the mandrel, any loose fibers or particles are blown off of the exterior of the shell with an anti-static air gun. A tack coat layer is then applied. The tack coat layer may be sprayed on, but is desirably applied by dipping the flexible shell on the mandrel into a tack coat dispersion. The operator immerses the flexible shell into the dispersion and returns the mandrel to a rack for stabilization. The time required for stabilization typically varies between 5-20 minutes. A suitable tack coat layer is desirably made using the same material employed in the base layers.

At this point, granulated solid particles (i.e., salt crystals) are applied over that portion of the exterior surface that will end up as the fixation surface. The solid particles may be applied manually by sprinkling them over the surface while the mandrel is manipulated, or a machine operating like a bead blaster or sand blaster could be used to deliver a steady stream of solid particles at an adequate velocity to the coating on the mandrel. However, a preferred method of solid particle application is to dip the mandrel/shell into a body of the solid particles or expose it to a suspension of the solid particles. It should be understood that the present invention is not intended to be restricted to any one particular method of applying particles, though care must be taken to ensure that the solid particles only adhere to the areas desired. One possible method to apply solid particles to some but not all of the implant is to mask off the other areas.

The tacky flexible shell is then immersed in a fluidized (air-mixing) aqueous salt bath having regular cubic salt crystals between about 10 to about 600 microns, or round crystals between 50-2000 microns. Varying degrees of texturing may be formed with the salt removal process by using differently sized or shaped salt granules (for example, round salt crystals versus angular salt crystals, large salt crystals versus relatively small salt crystals, high density distribution of salt crystals versus relatively low density distribution of salt crystals), on different areas of the shell. The shell is rotated for even coverage, removed, and then allowed to stabilize. After a suitable period of stabilization, such as between 5-20 minutes, the flexible shells may be dipped into an overcoat dispersion. A suitable overcoat dispersion is also desirably made using the same material employed in the base layers. The flexible shells on the mandrels are then mounted on a rack and allowed to volatilize, such as for example 15 minutes.

The entire silicone elastomer shell structure is vulcanized or cured in an oven at elevated temperatures. The temperature of the oven is preferably kept between about 39.3°C (200°F) and about 177°C (350°F) for a curing time preferably between about 20 minutes and about 1 hour, 40 minutes. Upon removal from the oven, the mandrel/shell assembly is placed in a solvent for the solid particles, and the solid particles allowed to dissolve. The solvent does not affect the structure or integrity of the silicone elastomer. When the solid particles have dissolved, the assembly is removed from the solvent and the solvent evaporated. The shell can then be stripped from the mandrel. At this point, it is preferable to place the shell in a solvent for the solid particles and gently agitate it to ensure complete dissolution of all the solid particles. When the shell is removed from the solvent, the solvent is evaporated.

Dissolving the solid particles leaves behind open spaces in the surface of the shell where the salt was applied. When applied, some of the solid particles are partially exposed so that they can be acted upon by the solvent. These exposed solid particles also provide a way for the solvent to reach those solid particles beneath the surface to dissolve them in turn. The result is an interconnected structure of cells, some of which are open to the surface, in the outer layer of the shell. The shell has a thin outer wall made of silicone elastomer with an opening therein at the point where a support member connected to the mandrel, which opening will subsequently be covered with a patch.

After finishing the shell according to the steps described above, the steps required to make a finished breast implant prosthesis are again similar to those used by other manufacturers. First, the opening left by the dip molding process is patched with unvulcanized sheeting, usually made of silicone rubber. Then, if the prosthesis is to be filled with silicone gel, this gel is added and cured, the filled prosthesis packaged, and the packaged prosthesis sterilized. If the prosthesis is to be inflated with a saline solution, a one-way valve is assembled and installed, the prosthesis is post cured if required, and the prosthesis is then cleaned, packaged and sterilized. A combination breast implant prosthesis can also be made wherein a gel-filled sac is positioned inside the shell to be surrounded by saline solution.

In addition to the aforementioned dipping process, the flexible shell for the prosthetic implant may be formed using a molding process. For example, a rotational molding process such as described in Schuessler, U.S. Patent No. 6,602,452, may be used. The process for forming texturing on the exterior surface may be done using a dipping technique after the shell is molded, but another method is to roughen the inside of the mold. For example, a mold having a generally smooth interior surface except for rough areas as described above will produce an implant shell having discrete fixation surfaces. The rotational molding process is advantageous because the entire implant shell may be formed in relatively few manufacturing steps.

Although the invention has been described and illustrated with a certain degree of particularity, it is understood that the present disclosure has been made only by way of example, and that numerous changes in the combination and arrangement of parts can be resorted to by those skilled in the art without departing from the scope of the invention, as hereinafter claimed.

## Claims

1. A breast prosthesis for implantation in a human being, the device comprising
a shell (112; 212) having an exterior structured to contact tissue, the shell including a first fixation surface (214) having a first open cell structure (216), comprising open cells, and a second fixation surface (218) having a second open cell structure (222), comprising open cells, different than said first open cell structure (216);
the first fixation surface (214) and the second fixation surface (218) being positioned to encourage respectively different degrees of tissue ingrowth or tissue adhesion by the body at a body-shell interface,
**characterized in that**
the open cells of the second open cell structure (222) are smaller than the open cells of the first open cell structure (216) .

2. The prosthesis of claim 1, wherein the first open cell structure (216) comprises a first distribution of cells and the second open cell structure (222) comprises a second distribution of cells wherein the first distribution of cells is relatively more dense than the second distribution of cells.

3. The prosthesis of claim 1, wherein the first open cell structure (216) comprises relatively large rounded open cells and the second open cell structure (222) comprises relatively small rounded open cells.

4. The prosthesis of claim 1, wherein the first open cell structure (216) comprises relatively rounded open cells and the second open cell structure (222) comprises relatively angular open cells.

5. The prosthesis of any of claims 1 to 4 further comprising a gel core enveloped by the shell (112; 212).

6. The prosthesis of any of claims 1 to 5, wherein the shell (112; 212) is fillable with saline after implantation in the body.

7. A breast prosthesis shell for implantation in a human being, the shell manufactured by the steps of:
(a) providing a shell precursor;
(b) applying a layer of silicone elastomer to the shell precursor;
(c) applying solid particles of a first configuration to a portion of the layer of silicone elastomer before the layer is fully cured;
(d) applying solid particles of a second configuration to another portion of the layer of silicone elastomer before the layer is fully cured, the second configuration being different from the first configuration; and
(e) fully curing the layer; and
(f) dissolving the solid particles with a solvent that does not dissolve the silicone elastomer to any appreciable extent to thereby form an elastomer shell having a first open cell texture region (216), comprising first open cells, formed by said application of the solid particles of the first configuration, and a second open cell texture region (222), comprising second open cells, formed by said application of the solid particles of the second configuration,
**characterized in that**
the open cells of the second open cell texture region (222) are smaller than the open cells of the first open cell texture region (216).

8. The prosthesis of claim 7 structured such that tissue adhesion or ingrowth by the body engages the first open cell texture region (216) differently than the second open cell texture region (222).

## Patentansprüche

1. Brustprothese zur Implantation in einen Menschen, wobei die Vorrichtung umfasst
eine Schale (112; 212), die eine Außenseite aufweist, die für Kontakt mit Gewebe strukturiert ist, wobei die Schale eine erste Fixierfläche (214) beinhaltet, die eine erste offene Zellstruktur (216) aufweist, die offene Zellen umfasst, und eine zweite Fixierfläche (218), die eine zweite offene Zellstruktur (222) aufweist, die offene Zellen umfasst und sich von der ersten offenen Zellstruktur (216) unterscheidet;
die erste Fixierfläche (214) und die zweite Fixierfläche (218) positioniert sind, jeweils unterschiedliche Grade von Gewebeeinwachsen oder Gewebeanhaftung durch den Körper bei einer Körper-Schalen-Übergangsstelle zu begünstigen,
**dadurch gekennzeichnet, dass**
die offenen Zellen der zweiten offenen Zellstruktur (222) kleiner als die offenen Zellen der ersten offenen Zellstruktur (216) sind.

2. Prothese nach Anspruch 1, wobei die erste offene Zellstruktur (216) eine erste Verteilung von Zellen umfasst und die zweite offene Zellstruktur (222) eine zweite Verteilung von Zellen umfasst, wobei die erste Verteilung von Zellen relativ dichter als die zweite Verteilung von Zellen ist.

3. Prothese nach Anspruch 1, wobei die erste offene Zellstruktur (216) relativ große, runde offene Zellen umfasst und die zweite offene Zellstruktur (222) relativ kleine, runde offene Zellen umfasst.

4. Prothese nach Anspruch 1, wobei die erste offene Zellstruktur (216) relativ runde offene Zellen umfasst und die zweite offene Zellstruktur (222) relativ eckige offene Zellen umfasst.

5. Prothese nach einem der Ansprüche 1 bis 4, weiter umfassend einen Gelkern, der von der Schale (112; 212) umhüllt wird.

6. Prothese nach einem der Ansprüche 1 bis 5, wobei die Schale (112; 212) nach Implantation in den Körper mit Kochsalzlösung befüllt werden kann.

7. Brustprothesenschale zur Implantation in einen Menschen, wobei die Schale durch die Schritte hergestellt wird vom:
a) Bereitstellen eines Schalenvorläufers;
b) Aufbringen einer Schicht aus Silikonelastomer auf den Schalenvorläufer;
c) Aufbringen festerTeilchen einer ersten Konfiguration auf einen Abschnitt der Schicht aus Silikonelastomer, bevor die Schicht vollständig ausgehärtet ist;
d) Aufbringen fester Teilchen einer zweiten Konfiguration auf einen anderen Abschnitt der Schicht aus Silikonelastomer, bevor die Schicht vollständig ausgehärtet ist, wobei die zweite Konfiguration sich von der ersten Konfiguration unterscheidet; und
e) vollständiges Aushärten der Schicht; und
f) Auflösen der festen Teilchen mit einem Lösemittel, das das Silikonelastomer nicht bis zu einem nennenswerten Ausmaß auflöst, um dadurch eine Elastomerschale zu bilden, die einen ersten offenen Zelltexturbereich (216), der erste offene Zellen umfasst, gebildet durch die Aufbringung der festen Teilchen der ersten Konfiguration, und einen zweiten offenen Zelltexturbereich (222), der zweite offene Zellen umfasst, gebildet durch die Aufbringung der festen Teilchen der zweiten Konfiguration, aufweist,
**dadurch gekennzeichnet, dass**
die offenen Zellen des zweiten offenen Zelltexturbereichs (222) kleiner als die offenen Zellen des ersten offenen Zelltexturbereichs (216) sind.

8. Prothese nach Anspruch 7, die so strukturiert ist, dass Gewebeanhaftung oder Einwachsen durch den Körper in den ersten offenen Zelltexturbereich (216) anders als in den zweiten offenen Zelltexturbereich (222) eingreift.

## Revendications

1. Prothèse mammaire pour l'implantation dans un être humain, le dispositif comprenant
une enveloppe (112 ; 212) présentant un extérieur structuré pour entrer en contact avec un tissu, l'enveloppe incluant une première surface de fixation (214) présentant une première structure de cellules ouvertes (216), comprenant des cellules ouvertes, et une seconde surface de fixation (218) présentant une seconde structure de cellules ouvertes (222), comprenant des cellules ouvertes, différente de ladite première structure de cellules ouvertes (216) ;
la première surface de fixation (214) et la seconde surface de fixation (218) étant positionnées pour favoriser respectivement différents degrés de croissance interne de tissu ou d'adhérence de tissu par le corps au niveau d'une interface corps-enveloppe,
**caractérisée en ce que**
les cellules ouvertes de la seconde structure de cellules ouvertes (222) sont plus petites que les cellules ouvertes de la première structure de cellules ouvertes (216).

2. Prothèse selon la revendication 1, dans laquelle la première structure de cellules ouvertes (216) comprend une première distribution de cellules et la seconde structure de cellules ouvertes (222) comprend une seconde distribution de cellules dans laquelle la première distribution de cellules est relativement plus dense que la seconde distribution de cellules.

3. Prothèse selon la revendication 1, dans laquelle la première structure de cellules ouvertes (216) comprend des cellules ouvertes arrondies relativement grandes et la seconde structure de cellules ouvertes (222) comprend des cellules ouvertes arrondies relativement petites.

4. Prothèse selon la revendication 1, dans laquelle la première structure de cellules ouvertes (216) comprend des cellules ouvertes relativement arrondies et la seconde structure de cellules ouvertes (222) comprend des cellules ouvertes relativement angulaires.

5. Prothèse selon l'une quelconque des revendications 1 à 4, comprenant en outre un noyau en gel enveloppé par l'enveloppe (112; 212).

6. Prothèse selon l'une quelconque des revendications 1 à 5, dans laquelle l'enveloppe (112; 212) peut être remplie de sérum physiologique après l'implantation dans le corps.

7. Enveloppe de prothèse mammaire pour l'implantation dans un être humain, l'enveloppe étant fabriquée par les étapes consistant à :
(a) fournir un précurseur d'enveloppe ;
(b) appliquer une couche d'élastomère de silicone au précurseur d'enveloppe ;
(c) appliquer des particules solides d'une première configuration à une partie de la couche d'élastomère de silicone avant que la couche ne soit entièrement durcie ;
(d) appliquer des particules solides d'une seconde configuration à une autre partie de la couche d'élastomère de silicone avant que la couche ne soit entièrement durcie, la seconde configuration étant différente de la première configuration ; et
(e) faire durcir entièrement la couche ; et
(f) dissoudre les particules solides avec un solvant qui ne dissout pas l'élastomère de silicone dans une mesure appréciable pour ainsi former une enveloppe d'élastomère présentant une première région de texture de cellules ouvertes (216), comprenant des premières cellules ouvertes, formée par ladite application des particules solides de la première configuration, et une seconde région de texture de cellules ouvertes (222), comprenant des secondes cellules ouvertes, formée par ladite application des particules solides de la seconde configuration,
**caractérisée en ce que**
les cellules ouvertes de la seconde région de texture de cellules ouvertes (222) sont plus petites que les cellules ouvertes de la première région de texture de cellules ouvertes (216).

8. Prothèse selon la revendication 7, structurée de sorte qu'une adhérence ou une croissance interne de tissu par le corps engage la première région de texture de cellules ouvertes (216) différemment de la seconde région de texture de cellules ouvertes (222).
